# EUROPEAN PATENT APPLICATION

(11) **EP 4 759 250 A2**
(43) Date of publication of application: **17.06.2026**
(21) Application number: 25460026.5
(22) Date of filing: 25.06.2025
(51) Int. Cl.: A61B 17/68, A61B 17/064

(54) **DISTRACTION EXPANDER AND METHOD FOR ADJUSTING THE FORCE OF A DISTRACTION EXPANDER FOR THE TREATMENT OF CRANIOSYNOSTOSIS**

(30) Priority: 15.12.2024 PL 45058324
(71) Applicant: Politechnika Czestochowska, 42-200 Czestochowa (PL)
(72) Inventor: Szarek, Arkadiusz, 42-218 Czestochowa (PL); Larysz, Dawid, 40-748 Katowice (PL); Larysz, Patrycja, 40-478 Katowice (PL)

(57) **Abstract**

The object of the invention is a cranial spring for the treatment of premature cranial suture fusion and a method for adjusting the force of the cranial spring.

The cranial spring is made of wire with a diameter of 0.2 mm to 3 mm and has two arms: a first arm **(1),** a second arm **(2),** a flexible bend **(3) ,** and clamps **(5)** located at the ends of both the first **(1)** and second arm **(2). It is characterised** by the presence of adjustment coils **(4)** on the first **(1)** and/or second arm **(2)** , while the flexible bend **(3)** is designed in such a way that the arms can be positioned relative to each other within a range of 0 to 120⁰ degrees

The method of adjusting the force in the cranial spring as described in claim **1** - 6 is **characterised in that** the force is obtained by stretching the adjustment coils **(4)** in the first arm **(1)** and/or the second arm **(2).**

## Description

### FIELD OF TECHNOLOGY

The object of the invention is a cranial spring for the treatment of premature cranial suture fusion and a method for adjusting the force of the cranial spring.

### STATE OF THE ART

Craniosynostosis, the premature fusion of one or more cranial sutures, is a rare congenital disorder. The only effective treatment is surgery involving the reconstruction of the cranial bones. All types of craniosynostosis, both syndromic (including Crouzon syndrome, Apert syndrome, Saethre-Chotzen syndrome, Muenke syndrome, Pfeiffer syndrome, and others) and non-syndromic (including sagittal, bilateral and unilateral coronal, metopic, lambdoid, and rare craniosynostoses of other sutures), can be treated with minimally invasive procedures at the appropriate age. One group of minimally invasive procedures available are cranial reconstruction procedures using dedicated cranial springs (spring assisted surgery - SAS). The use of this method allows for a reduction of surgery time, the extent of the procedure and blood loss. An extension of the above methods is the use of endoscopically implanted cranial springs - ESAS surgery.

One of the first techniques used in minimally invasive cranial reconstruction surgery in children with craniosynostosis was endoscopic suturectomy, followed by the need for postoperative head shaping using a customised helmet-type orthosis [1, 2]. Subsequently, several types of minimally invasive endoscopic reconstructive procedures for direct skull remodelling have emerged, reducing or completely eliminating the orthotic therapy that was previously considered the basis of treatment [3, 4, 5].

The correction of sagittal craniosynostosis using cranial springs was introduced by Lauritzen et al. The authors were the first to describe a case of treatment using this method in 1998 [6]. This technique usually consists of a craniotomy or a sagittal craniectomy, followed by the insertion of a series of internal springs perpendicular to the prematurely fused suture. The expansion force of the springs gradually changes the shape of the skull, increasing its frontal area. After a period of re-ossification, the springs must be removed. Now, more than a decade after its introduction, this technique is being used on a wider scale [7, 8].

The cranial springs currently available on the market are characterised by a constant distraction force, which in many cases is a serious limitation for this treatment method.

Spring-assisted cranioplasty can be performed using an endoscope. The achieved cranial correction effect, e.g. with regard to the cranial index, is at least comparable to conventional methods (SAS). The ESAS procedure requires only one or two small incisions in the cranial shell. This reduces blood loss, the duration of the surgery and the incidence of complications. The main disadvantage of this method is the need for a second surgery to remove the springs. The second disadvantage is that, once the spring has been placed in the skull, its expansion cannot be controlled. The use of customised bone cutting line planning and customised springs can largely eliminate or reduce the aforementioned problems [9].

The knowledge of the elastic modulus and thickness of the cranial vault bones (based on a preoperative CT scan of the head) allows the prediction of changes in head shape and cranial index after reconstruction of the skull via a spring in sagittal craniosynostosis [10].

Current solutions are based on springs with a constant distraction force, the value of which depends on the type of distractor material, wire thickness, arm length and the design, in which the distractor is a V-shaped rod or a V-shaped rod with one circular coil in the area of the bend [11].

### INVENTION DISCLOSURE

The technical problem solved by the invention is the regulation of the distraction force, which is achieved by lengthening or shortening the arm of the cranial spring, thus increasing the arm and the distraction torque.

In addition, there is a technical problem with the way the force is regulated in the cranial spring, which is solved by increasing or decreasing the length of the spring arm.

In light of the described state of the art, the aim of this invention is to overcome the indicated inconveniences and provide a device with adjustable distraction force, where by adjusting the length of the arm, with the same tension force, the torque of the force changes by increasing the length of the arm.

The invention also aims to regulate the force by changing the length of the arm in the cranial spring.

The cranial spring is made of wire with a diameter of 0.2 mm to 3 mm and has two arms: a first arm, a second arm, a flexible bend, and the ends of the first and second arm. It is characterised by the presence of adjustment coils on the first and/or second arm, while the flexible bend is designed in such a way that the arms can be positioned relative to each other within a range of 0 to 120⁰ degrees

Its advantage stems from the fact that it is made of a biologically compatible, highly biocompatible and resilient material.

Furthermore, it is made using the drawing and/or bending and/or 3D printing technique, and its cross-section is round or polygonal.

Its adjustment coils are in the form of a sine wave, and/or circular coils, and/or trapezoidal coils, and/or triangular coils, and/or square coils, with the amplitude range of the wave being between one and fifty times the diameter of the wire, while the spacing between the waves is between one wire diameter and twenty times the wire diameter.

Preferably, the ends are in the form of hooks and/or round lugs and/or lugs with right angles and/or round swaged lugs and/or triangular swaged lugs and/or inverted triangular swaged lugs and/or square swaged lugs.

A spring has individual parts made of a single material and/or different materials.

The method of force adjustment in the cranial spring, as described above, relies on obtaining the desired force by stretching the adjustment coils in the first and/or second arm.

The advantage of the solution used is its simplicity and adaptability, i.e. one type of cranial spring can be used for different cases, and the desired tension force can be achieved through adjustment.

Another advantage is the reduction in the number of spring types, which can be used for many cases thanks to the adjustment fitted.

An important advantage is that the lateral arms consisting of a flat coil with a sinusoidal form, different shape, size, number and height of coils, are adjustable, i.e. thanks to these parameters, the range of forces to be generated by the spring is obtained.

### BRIEF DESCRIPTION OF THE FIGURES

In order to facilitate understanding of the invention, it has been illustrated in the following embodiments and in the attached drawing figures:
**Fig. 1** **-** shows a view of the cranial spring divided into sections.
**Fig. 2** **-** shows a view of the cranial spring with circular coils.
**Fig. 3** **-** shows a view of a cranial spring with trapezoidal coils.
**Fig. 4** **-** shows a view of a cranial spring with triangular coils.
**Fig. 5** **-** shows a view of a cranial spring with square coils.
**Fig. 6** **-** shows a view of the cranial spring with circular coils, a bending radius equal to the wire diameter, and a coil height equal to the wire diameter.
**Fig. 7** **-** shows a view of a distraction expander with round coils and a bending radius equal to the wire diameter and a coil height equal to twice the wire diameter.
**Fig. 8** **-** shows a view of a distraction expander with round coils and a bending radius equal to the wire diameter and a coil height equal to 10 times the wire diameter.
**Fig. 9** **-** shows a view of a distraction expander with round coils and a bending radius equal to the 10 times the wire diameter and a coil height equal to 10 times the wire diameter.
**Fig. 10** **-** shows a view of the arm length adjustment to achieve the desired distraction force.
   **A)** Asymmetrical;
   **B)** Asymmetrical.
**Fig. 11****-** shows a view of the lugs:
   **A)** round lug;
   **B)** lug with right angles;
   **C)** round swaged lug;
   **D)** triangular swaged lug;
   **E)** inverted triangular swaged lug;
   **F)** square swaged lug.
~ **Fig. 12** **-** shows a square lug perpendicular to the plane of the circular coil stretched to the desired distraction force.

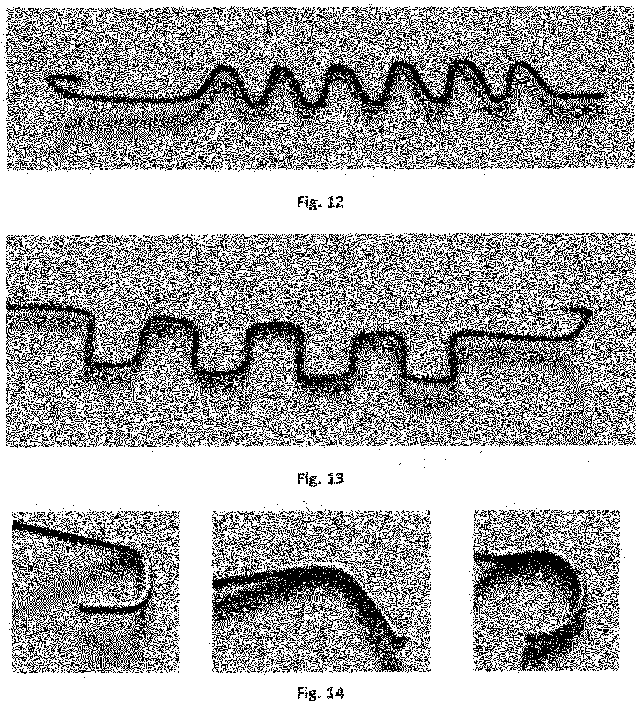

**Fig. 13** **-** shows a square lug perpendicular to the plane of the square coil stretched to the desired distraction force.
**Fig. 14** **-** shows a view of example lug shapes.

The cranial spring and the method of force adjustment shown in the attached figures are for illustrative purposes only. The final shape, along with the applied adjustment coils, will depend on the specific case.

### EMBODIMENTS

The following examples illustrate the invention without limiting its scope in any way.

### Example 1. CRANIAL SPRING

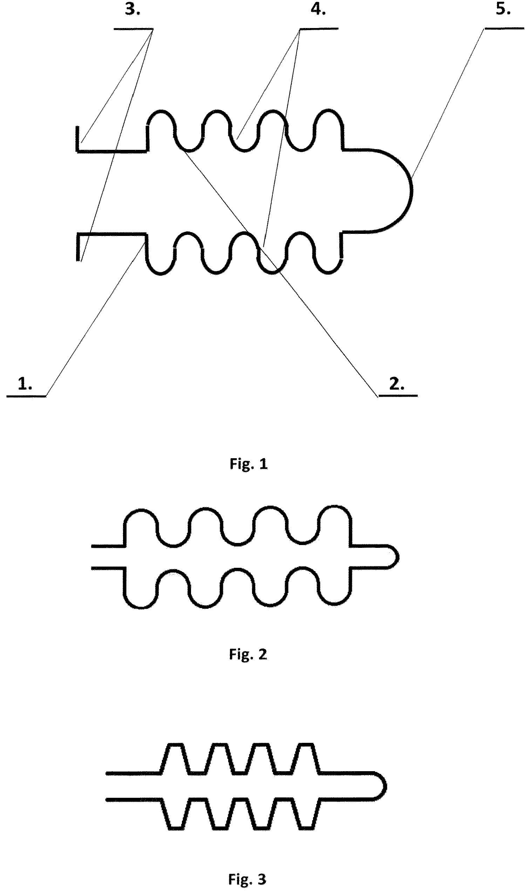

**Figure 1** **shows cranial spring made of wire with a diameter of 1.5 mm.**

The spring has two arms, i.e. first arm **(1),** second arm **(2),** flexible bend **(3)** and ends **(5),** with first arm **(1)** and second arm **(2)** having adjustment coils **(4),** with a structure resembling a circular coil, which is shown in Fig. 2.

The cranial spring is made of a biologically compatible material with high biotolerance and elastic properties, manufactured using the cold-drawing method. The wire has a circular cross-section.

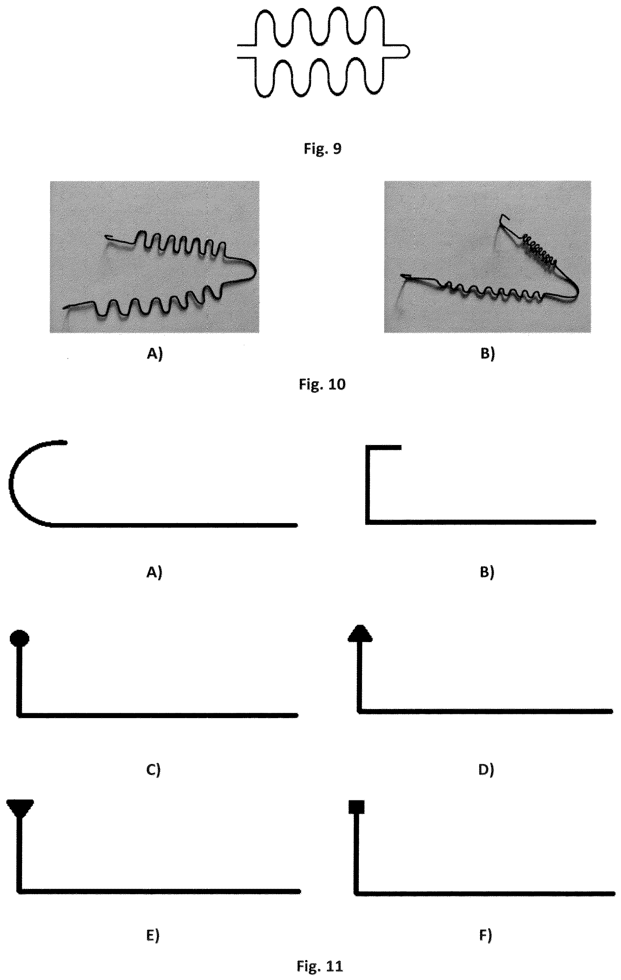

The ends of the first **(1)** and the second arm **(2)** include round lugs, as shown in **Fig. 11A** ).

### Example 2. METHOD OF FORCE ADJUSTMENT IN A CRANIAL SPRING

The method of force adjustment in the cranial spring, as described in **Example 1,** involves achieving the desired force by stretching the adjustment coils **(4)** in the first arm **(1)** and the second arm **(2).**

The cranial spring is secured within the bone undergoing distraction in a specially prepared bone recess, with a diameter no smaller than the thickness of the spring. Stabilisation within the bone is achieved through a specially designed system of hooks or round (swaged) attachments of various shapes. The increase in the spring's force (torque) is achieved by stretching the flat winding to the desired distraction force.

The extension of the spring's arms is accomplished by stretching the flat coil to the required length.

The adjustment method is illustrated in **Figure 10****.**

### Example 3. CRANIAL SPRING

The cranial spring has the same structure as in **Example 1,** but the wire diameter is 1.7 mm, and the adjustment coils (**4**) have a trapezoidal shape, as shown in **Figure 3****.**

The ends have the shape of lugs with right angles, as shown in Fig. 11 B).

### Example 4. CRANIAL SPRING

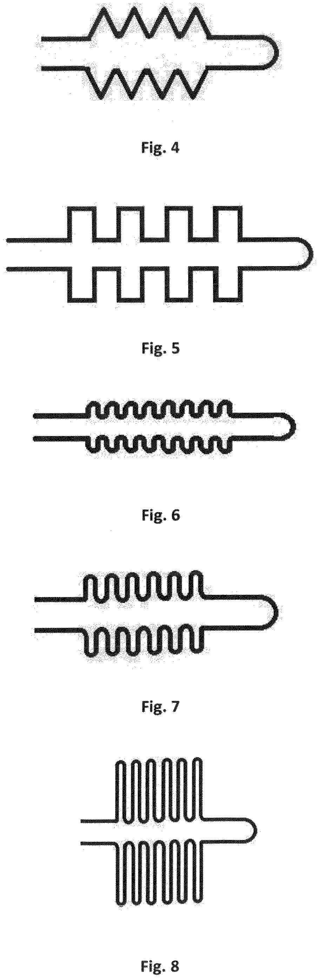

The cranial spring has the same structure as in **Example 1,** but the wire diameter is 2.0 mm, and the adjustment coils (**4**) have a triangular shape, as shown in **Figure 4****.**

The ends have the shape of round swaged lugs, as shown in Fig. 11 C).

### Example 5. CRANIAL SPRING

The cranial spring has the same structure as in **Example 1,** but the wire diameter is 2.0 mm, and the adjustment coils (**4**) have a square shape, as shown in **Figure 5****.**

The ends have the shape of triangular swaged lugs as shown in Fig. 11 D).

### Example 6. CRANIAL SPRING

The cranial spring has the same structure as in **Example 1,** but the wire diameter is 2.0 mm. The adjustment coils (**4**) have a circular shape, with a bending radius equal to the wire diameter and a coil height equal to the wire diameter, as shown in **Figure 6****.**

The ends have the shape of inverted triangular swaged lugs as shown in Fig. 11 E).

### Example 7. CRANIAL SPRING

The cranial spring has the same structure as in **Example 1,** but the wire diameter is 2.0 mm. The adjustment coils (**4**) have a circular shape, with a bending radius equal to the wire diameter and a coil height equal to twice the wire diameter, as shown in **Figure 7****.**

The ends have the shape of square swaged lugs as shown in Fig. 11 F).

### Example 8. CRANIAL SPRING

The cranial spring has the same structure as in **Example 1,** but the wire diameter is 2.0 mm. The adjustment coils (**4**) have a circular shape, with a bending radius equal to the wire diameter and a coil height equal to the wire diameter, as shown in **Figure 8****.**

The ends have the shape of round swaged lugs, as shown in Fig. 11 C).

### Example 9. CRANIAL SPRING

The cranial spring has the same structure as in **Example 1,** but the wire diameter is 2.0 mm. The adjustment coils (**4**) have a circular shape, with a bending radius equal to ten times the wire diameter and a coil height equal to ten times the wire diameter, as shown in **Figure 9****.**

The ends have the shape of round swaged lugs, as shown in Fig. 11 C)

### REFERENCES:

**1.** Jimenez DF, Barone CM (1998) Endoscopic craniectomy for early surgical correction of sagittal craniosynostosis. J Neurosurg 88:77-81.
**2.** Jimenez DF, Barone CM (2012) Endoscopic technique for sagittal synostosis. Child's Nervous System : Chns : Official Journal of the International Society for Pediatric Neurosurgery, 08 Aug 2012, 28(9):1333-1339.
**3.** Di Rocco C (2003) How to decrease the impact of surgical scar in the correction of sagittal synostosis. Childs Nerv Syst (2003) 19:42-45.
**4.** Massimi L, Di Rocco C (2012) Mini-invasive surgical technique for sagittal craniosynostosis. Childs Nerv Syst. 2012 Sep;28(9):1341-5.
**5.** Mutchnick IS, Maugans TA (2012) Nonendoscopic, minimally invasive calvarial vault remodeling without postoperative helmeting for sagittal synostosis Clinical article. J Neurosurg Pediatr 9(3):222-227.
**6.** Lauritzen C, Sugawara Y, Kocabalkan O. et al: Spring-mediated dynamic craniofacial reshaping. Scand J Plast Reconstr Hand Surg 32:331-33.
**7.** Taylor Jesse Adam, Maugans, Todd A.: Comparison of Spring-Mediated Cranioplasty to Minimally Invasive Strip Craniectomy and Barrel Staving for Early Treatment of Sagittal Craniosynostosis, Journal of Craniofacial Surgery 22(4):p 1225-1229, July 2011.
**8.** David, Lisa R.; Plikaitis, Christina M.; Couture, Daniel; Glazier, Steven S.; Argenta, Louis C.: Outcome Analysis of Our First 75 Spring-Assisted Surgeries for Scaphocephaly. Journal of Craniofacial Surgery 21(1):p 3-9, January 2010.
**9.** Marie-Lise C. van Veelen & Irene M. J. Mathijssen, Spring-assisted correction of sagittal suture synostosis, Childs Nerv Syst (2012) 28:1347-1351.
**10.** Dillan F. Villavisanis, Daniel Y. Cho, Chao Zhao, Connor S. Wagner, Jessica D. Blum, Sameer Shakir, Jordan W. Swanson, Scott P. Bartlett, Alexander M. Tucker & Jesse A. Taylor, Spring forces and calvarial thickness predict cephalic index changes following spring-mediated cranioplasty for sagittal craniosynostosis, Child's Nervous System (2023) 39:701-709.
**11.** KLS Martin Group, CranioXpand Cranial springs for the treatment of sagittal craniosynostoses, 90-264-02-04 · Rev. 02 · 2023-11 · Printed in Germany · Copyright by KLS Martin SE & Co. KG · All rights reserved · Subject to technical changes 8, (1998).

### LIST OF DESIGNATIONS:

**1.** first arm;
**2.** second arm;
**3.** flexible bend;
**4.** adjusment coils;
**5.** ends.

## Claims

1. The cranial spring is made of wire with a diameter of 0.2 mm to 3 mm and has two arms: a first arm (**1**), a second arm (**2**), a flexible bend (**3**), and clamps (**5**) located at the ends of both the first (**1**) and second arm (**2**). **It is characterised by** the presence of adjustment coils (**4**) on 5 the first (**1**) and/or second arm (**2**) , while the flexible bend (**3**) is designed in such a way that the arms can be positioned relative to each other within a range of 0 to 120⁰ degrees

2. Cranial spring according to claim 1 **is characterised in that it** is made of a biologically compatible, highly biocompatible and elastic material.

3. Cranial spring according to claims 1 - 2 **is characterised in that it** is made using the drawing 10 and/or bending and/or 3D printing technique, and its cross-section is round or polygonal.

4. Cranial spring according to claims 1 - 3 **is characterised in that** its adjustment coils (**4**) are in the form of a sine wave, and/or circular coils, and/or trapezoidal coils, and/or triangular coils, and/or square coils, with the amplitude range of the wave being between one and fifty times the diameter of the wire, while the spacing between the waves is between one wire diameter 15 and twenty times the wire diameter.

5. Cranial spring according to claims **1 - 4**is **characterised in that** its ends (**5** are in the form of hooks and/or round lugs and/or lugs with right angles and/or round swaged lugs and/or triangular swaged lugs and/or inverted triangular swaged lugs and/or square swaged lugs.

6. Cranial spring according to claims 1-5 **is characterised in that** the spring is made of a single 20 material and/or has individual parts made of different materials.

7. The method of adjusting the force in the cranial spring as described in claim 1 - 6 **is characterised in that** the force is obtained by stretching the adjustment coils (**4**) in the first arm (1) and/or the second arm (**2**).
